Europäisches Patentamt

European Patent Office

Office européen des brevets

(11) Publication number: **0 516 270 A2**

## EUROPEAN PATENT APPLICATION

(21) Application number: **92303186.8**

(22) Date of filing: **09.04.92**

(51) Int. Cl.5: **C12N 15/36**, C07K 15/00, G01N 33/576

A request for addition missing drawings has been filed pursuant to Rule 88 EPC. A decision on the request will be taken during the proceedings before the Examining Division (Guidelines for Examination in the EPO, A-V, 2.2).

(30) Priority: **10.04.91 JP 196175/91**

(43) Date of publication of application:
**02.12.92 Bulletin 92/49**

(84) Designated Contracting States:
**BE CH DE DK FR GB IT LI NL**

(71) Applicant: **IMMUNO JAPAN INC.**
**4-28-14-701, Ogikubo Suginami-Ku**
**Tokyo 167(JP)**

(72) Inventor: **Okamoto, Hiroaki**
**3132-24, Yakushi Ji, Minami Kawachi Machi**
**Kawachi-Gun, Tochigi-Ken(JP)**
Inventor: **Nakamura, Tetsuo**
**4-28-14-701, Ogikubo, Suginami-ku**
**Tokyo 167(JP)**

(74) Representative: **Arthur, Bryan Edward**
**Withers & Rogers 4 Dyer's Buildings Holborn**
**London EC1N 2JT(GB)**

(54) **Non-A, non-B hepatitis virus related antigen, antibody, detection systems, polynucleotides and polypeptides.**

(57) Non-A, non-B hepatitis (NANB hepatitis) virus RNA and its corresponding polypeptide, related antigen, antibody, and detection systems for detecting NANB hepatitis antigen or antibodies.

EP 0 516 270 A2

## Reference To A Related Application

The present application is a continuation-in-part of our copending U.S. Patent Application Serial No. 07/790,382, filed on November 8, 1991, which is incorporated by reference in its entirety.

## Background of the Invention

The present invention relates to non-A, non-B hepatitis (hereinafter called NANB hepatitis) virus RNA and its corresponding polypeptide, related antigen, antibody, and detection systems for detecting NANB hepatitis antigen or antibodies.

Viral hepatitis (of which the DNA and the RNA of the causative viruses have been elucidated and their diagnosis, and even prevention in some cases, have been established) is caused by hepatitis A, hepatitis B, hepatitis D and hepatitis E. In spite of great efforts by scientists the world over, however, the causative virus of NANB hepatitis (falling in none of the above categories and mainly caused by blood born infection) has not been isolated.

In 1988, Chiron Corp. claimed that they had succeeded in cloning the RNA virus genome, which they termed hepatitis C virus (hereinafter called HCV), as the causative agent of NANB hepatitis and reported on its nucleotide sequence (British Patent 2,212,511 which is the equivalent of European Patent Application 0,318,216). HCV antibody detection systems based on the sequence are now being introduced for screening of blood for transfusion and for diagnosis of patients. Detection systems for the HCV antibody have proven their partial association with NANB hepatitis; however, they capture only about 70% of carriers and chronic hepatitis patients, or they fail to detect the antibody in acute phase infection, thus leaving problems yet to be solved even after development of the HCV antibody by Chiron Corp.

On the other hand, detection methods for hepatitis B virus have been established. More than 95% of post-transfusion cases of hepatitis in Japan are caused by NANB hepatitis (with annual estimated cases of 280,000). The course of NANB hepatitis is troublesome and most patients are considered to become carriers, then to develop chronic hepatitis. In addition, those patients with chronic hepatitis develop liver cirrhosis, then hepatocellular carcinoma at fairly high rates over 10 to 20 years. It is therefore very imperative to isolate the virus itself and to develop effective diagnostic reagents enabling earlier diagnosis.

The presence of a number of NANB hepatitis cases which can not be diagnosed by Chiron's HCV antibody detection kits demands development of NANB hepatitis diagnostic kits of more specificity. To develop such kits, it becomes an absolutely important task to analyze the causative virus of NANB hepatitis at its genetic (and corresponding amino acid) level.

## Summary of the Invention

An object of this invention is to provide the nucleotide sequence coding for the structural protein of NANB hepatitis virus and, with such information, to analyze amino acids of the protein to locate and provide polypeptides useful as antigens for establishment of detection systems for NANB virus, its related antigens and antibodies.

A further object of the present invention is to locate polypeptides effective as antigens by isolating NANB hepatitis virus RNA from human and chimpanzee virus carriers, cloning the cDNA covering the whole structural gene of the virus to determine its nucleotide sequence, and studying the amino acid sequence of the cDNA. As a result, a specific region of the structural gene of the virus coding for the core protein and envelope protein and the non-structural gene coding for RNA-dependent polymerase were determined.

## Brief Description of the Drawings

Figure 1(a), 1(b), 1(c), 1(d), and 1(e) represent restriction maps of the nucleotide sequences of 5, termini of NANB hepatitis virus strains HC-J1, HC-J4, HC-J5, HC-J6 and HC-J7 respectively; figure 1(f) shows the method of determination of the nucleotide sequence of 3' terminus of the strains. Solid lines show nucleotide sequences determined by clones from libraries of bacteriophage lambda gt10, and broken lines show nucleotide sequences determined by clones obtained by PCR.

Figure 2 is a plot of hydrophilicity of amino acids of the structural protein of NANB hepatitis virus.

Figure 3 is a graph showing the time-course of ALT, anti-CP9, and anti-HCV levels in a post-transfusion NANB hepatitis patient.

## Detailed Description of the Invention

Abbreviations used in the present invention are as follows: For RNA, A, G, C and U stand for Adenine, Guanine, Cytosine and Uracil respectively. For DNA, A, G and C indicate the same bases as in RNA and T stands for Thymine. For polypeptides, A, R, N, D, C, E, Q, G, H, I, L, K, M, F, P, S, T, W, Y and V show the respective amino acids Alanine, Arginine, Asparagine, Aspartic Acid, Cysteine, Glutamic Acid, Glutamine, Glycine, Histidine, Isoleucine, Leucine, Lysine, Methionine, Phenylalanine, Proline, Serine, Threonine, Tryptophan, Tyrosine and Valine.

In the method described below, NANB hepatitis virus RNA under this invention was obtained and its nucleotide sequence was determined.

Five samples (HC-J1, HC-J4, HC-J5, HC-J6, and HC-J7) were obtained from human and chimpanzee plasma. HC-J1, HC-J5, and HC-J6 were obtained from Japanese blood donors, and HC-J7 from a Japanese hemodialysis patient with CRF (chronic renal failure), all of whom tested positive for HCV antibody. HC-J4 was obtained from the chimpanzee subjected to the challenge test but was negative for Chiron's HCV antibody previously mentioned.

RNA was isolated from each of the five plasma samples and the homology of the nucleotide sequences were compared with each other. The homology of the nucleotide sequences were compared with each other and the respective nucleotide sequences of about 1900 to 2500 nucleotides of the 5' terminus and about 1100 nucleotides of the 3' terminus were determined (as described in the examples). The present inventors have completely studied the non-coding region, the regions coding for structural protein, and the partial regions coding for non-structural protein of all five strains.

As shown in Example 1, strains HC-J1, HC-J4, HC-J5, HC-J6 and HC-J7 had a 5' region consisting of 1885 to 2551 nucleotides that contains the noncoding region consisting of at least 340 or 341 nucleotides and a region coding for the structural protein (1149 nucleotides) followed by a region coding for the non-structural protein (see sequence lists 1-5).

Concerning the 3' terminus and 5' terminus, as described in example 3, HC-J1, HC-J4, HC-J5, HC-J6 and HC-J7 were found to have a region consisting of 1096 nucleotides coding for the non-structural protein followed by non-coding region consisting of at least 77 nucleotides that contains the T-stretch of 3' terminus. The 3' terminal nucleotides are shown in sequence lists 6-10.

When compared with the sequence of HCV disclosed in European Patent Application No. 88310922.5 (by Chiron Corp.), the homology of the sequences under this invention in comparison to HCV was 95.2% for strain HC-J1, 80.5% for strain HC-J4, 72.5% for strain HC-J6 and 71.6% for HC-J7 in the 2477 nucleotides of the 5' terminus including the non-coding region, and only 76.8% for HC-J7 in 1468 nucleotides. This result may suggest that strain HC-J1 may be relatively close to the strain determined by Chiron Corp. and thought to be an American type virus, but the others are different and are Japanese type viruses.

Based on the nucleotide sequences of strains HC-J1, HC-J4, HC-J5, HC-J6 and HC-J7, amino acid sequences of the coding region of the 5' terminus (sequence lists 11-15) and 3' terminus (sequence lists 16-20) respectively were determined: HC-J1 (733 amino acids, 365 amino acids), HC-J4 (733 amino acids, 365 amino acids), HC-J5 (515 amino acids, 365 amino acids), HC-J6 (737 amino acids, 365 amino acids), and HC-J7 (719 amino acids, 365 amino acids). From the amino acid sequences, polypeptides were obtained by synthesis.

When the amino acid sequences of strains HC-J1 and HC-J4 were compared, there was higher amino acid sequence homology in the upstream region than in the downstream region, and the upstream region was hydrophilic (containing more basic amino acids like arginine). Basic amino acids tend to be found in the core protein of various viruses providing affinity with nucleic acids; therefore, this upstream region was considered to code for the core protein while the downstream region (where frequent displacement of amino acids and as many as eight glycosylation sites were found) was considered to be coding for the surface protein.

Homology of the amino acid sequences of the 5' terminus of each strain was examined and divided into upper region and lower region. The homology of HC-J1 and HC-J4 was 96.9% and that of HC-J6 and HC-J7 was 95.3% for amino acids 1-191 throughout the region considered as core; however, homology between HC-J1 and HC-J6 and between HC-J4 and HC-J7 were only 90.6%. For amino acids 192-383 which is considered envelope protein, the homology was lower than that of the core region; the homology between HC-J1 and HC-J4 was 77.6% and between HC-J6 and HC-J7 was 71.4%; homology between HC-J1 and HC-J6 was 60.9% and between HC-J4 and HC-J7 was 52.6%.

The high homology of the amino acid sequence of the core region of each strain suggested the possibility of good detection of NANB hepatitis virus of both the American type and the Japanese type by an antibody detection system using polypeptides of the present invention.

As the result of examination of hydropathy profile, polypeptides designated in sequence lists 21-27 were selected and synthesized. Each polypeptide originated in strains HC-J1, HC-J4, HC-J5, HC-J6 or HC-J7. Peptides having partial displacement(s) in their amino acid sequence, yet retaining antigenicity, are included in the present invention.

Polypeptides having the sequences in sequence lists 21-27 were obtained by synthesis and detectability of antibody to NANB hepatitis with high specificity was confirmed using the polypeptides.

Obtaining protein from the envelope region is essential to development of a vaccine for protection against infection caused by NANB hepatitis virus. Thus the amino acid sequences of the Japanese type viruses of the present invention is very important, as well as Chiron's sequence, because homology of amino acid sequences suggested that the Japanese type NANB hepatitis virus could be distinguished from that of the American type virus and that some variants were observed in the Japanese types.

HCV is supposed to be closely related to flavivirus in regard to its genetic structure. The non-structural nucleotides of the 5' terminus of the present invention (nt 1491- ) is assumed to correspond to a region coding for glycoprotein called NS-1. Concerning yellow fever virus, which is a kind of flavivirus, Schlesinger et al. (J. Virology (1986), volume 60, page 1153) reported that antibody to the protein coded for by the NS-1 region was perhaps related to immunological protection. Useful proteins for treatment of and protection from NANB hepatitis will be able to be obtained by using the sequences of nucleotides and amino acids of the present invention.

The homology of the 3' terminal sequence (365 amino acids) was 87.4% between HC-J1 and HC-J4 and was 89.6% between HC-J6 and HC-J7; the homology between HC-J1 and HC-J6 was 73.7%. Homology for 39 nucleotides of the non-coding region was 64.1% for HC-J1 and HC-J4 and was 82.1% for HC-J6 and HC-J7; the homology between HC-J1 and HC-J6 was 30.8%. These results reflect the differences between the American type and Japanese type viruses and also variation among the Japanese types.

As pointed out above, HCV is supposed to be closely related to flavivirus in regard to its genetic structure. The non-structural nucleotides of the 3' terminus of the present invention is assumed to correspond to a region called NS-5 coding for RNA-dependent polymerase and thus has great importance in the diagnosis of NANB hepatitis via the detection of NANB antibody by using polypeptide coded for by the NS-5 region. In addition, the amino acid sequence of the polymerase obtained by study of the nucleotide sequence of the NS-5 region of both the American type and Japanese type viruses is very useful for treatment of and protection from NANB hepatitis virus.

It was confirmed that the regions coding for the structural protein and non-structural protein play an important role in the antigenicity of NANB hepatitis virus and the nucleotide sequence was clarified. Amino acid sequences constituting the structural protein and non-structural protein provide important material for searching for epitopes having antigenicity.

The polypeptides of the present invention can be obtained not only by chemical synthesis but by known recombinant technology as expressed peptides. Antibody to each polypeptide was made and its capacity to be utilized in the detection of NANB hepatitis virus antigen was confirmed.

A detection system using each polypeptide of the present invention or polypeptide with partial replacement of amino acids, and the detection system using antibody to such polypeptides, are useful as diagnostic agents of NANB hepatitis with high specificity and are effective to screen out NANB hepatitis virus from bloods for transfusion or blood derivatives. The polypeptides, or antibodies to such polypeptides, can be used a material for a vaccine against NANB hepatitis virus.

It is well known in the art that one or more nucleotides in a DNA sequence can be replaced by other nucleotides in order to produce the same protein. The present invention also concerns such nucleotide substitutions which yield DNA sequences which code for polypeptides as described above. It is also well known in the art that one or more amino acids in an amino acid sequence can be replaced by equivalent other amino acids, as demonstrated by U.S. Patent No. 4,737,487 which is incorporated by reference, in order to produce an analog of the amino acid sequence. Any analogs of the polypeptides of the present invention involving amino acid deletions, amino acid replacements, such as replacements by other amino acids, or by isosteres (modified amino acids that bear close structural and spatial similarity to protein amino acids), amino acid additions, or isosteres additions can be utilized, so long as the sequences elicit antibodies recognizing NANB antigens.

## Examples

Examples of this invention are shown below; however, this invention shall in no way be limited to those examples.

Example 1

5' terminal nucleotide sequence and amino acid sequence of NANB hepatitis virus genome were determined in the following way:

(1) Isolation of RNA

RNA of the sample (HC-J1, HC-J5, HC-J6) from plasma of a Japanese blood donor and that of the sample (HC-J7) from the plasma of the Japanese hemodialysis patient with CRF (who tested positive for HCV antibody by Ortho HCV An ELISA, Ortho Diagnostic System, Tokyo), and that of the sample (HC-J4) from the chimpanzee challenged with NANB hepatitis for infectivity (and negative for HCV antibody), were isolated in the following method.

1.8 ml of the plasma samples was added with 1 ml of Tris chloride buffer (10 mM, pH 8.0) and centrifuged at 68 x $10^3$ rpm for 1 hour. Its precipitate was suspended in Tris chloride buffer (50 mM, pH 8.0; containing 200 mM NaCl, 10 mM EDTA, 2% (w/v) sodium dodecyl sulfate (SDS), and 1 mg/ml proteinase K) and was incubated at 60½C for 1 hour. Then the nucleic acids were extracted by phenol/chloroform and precipitated by ethanol to obtain RNAs.

(2) cDNA Synthesis

After heating the RNA isolated from HC-J1 plasma at 70½C for 1 minute, this was used as a template; 10 units of reverse transcriptase (cDNA Synthesis System Plus, Amersham Japan) and 20 pmol of oligonucleotide primer (20 mer) were added and incubated at 42½C for 1.5 hours to obtain cDNA. Primer #8 (5'-GAT GCT TGC GGA AGC AAT CA-3') was prepared by referring to the basic sequence shown in European Patent Application No. 88310922.5, which is relied on and incorporated herein by reference.

(3) cDNA Was Amplified by the following Polymerase Chain Reaction (PCR)

cDNA was amplified for 35 cycles according to Saiki's method (Science (1988) 239: 487-491) using Gene Amp DNA Amplifier Reagent (Perkin-Elmer.Cetus) on a DNA Thermal Cycler (Perkin-Elmer.Cetus).

(4) Determination of 5' Terminal Nucleotide Sequence of HC-J1 and HC-J4 by Assembling cDNA Clones

As shown in Figure 1(a) and 1(b), nucleotide sequences of 5' termini of the genomes of strains HC-J1 and HC-J4 were determined by combined analysis of clones obtained from the cDNA library constructed in bacteriophage lambda gt10 and clones obtained by amplification of HCV specific cDNA by PCR. Fig. 1 shows 5' terminus of NANB hepatitis virus genome together with cleavage site by restriction endonuclease and sequence of primers used. In Fig. 1, solid lines are nucleotide sequences determined by clones from bacteriophage lambda gt10 library while dotted lines show sequences determined by clones obtained by PCR.

A 1656 nucleotide sequence of HC-J1 (spanning nt454-2109) was determined by clone Ú41 which was obtained by inserting the cDNA synthesized with the primer #8 into lambda gt10 phage vector (Amersham).

Primer #25 (5'-TCC CTG TTG CAT AGT T CAC G-3') of nt824-843 was synthesized based on the Ú41 sequence, and four clones (Ú60, Ú61, Ú66 and Ú75) were obtained to cover the upstream sequence nt18-843.

To determine the extreme upstream of the 5' terminus of HC-J4, single-stranded cDNA was synthesized using antisense primer #36 (5'-A ACA CTA CTC GGC TAG CAG T-3') of nt246-265, and then it was added with dATP tail at its 3' terminus by terminal deoxynucleotidyl transferase, then amplified by one-sided PCR in two stages. That is, in the first stage, oligo dT primer (20-mer) and antisense primer #48 (5'-GTT GAT CCA AGA AAG G ACC C-3') of nt188-207 were used to amplify the dA-tailed cDNA by PCR for 35 cycles; and in the second stage, using the product of the first-stage PCR as a template, oligo dT primer (20-mer) and antisense primer #109 (21-mer; 5'-ACC GGA TCC GCAGAC CAC TAT-3') of nt140-160 were added to initiate PCR for 30 cycles. The product of PCR was subcloned to M13 phage vector. Six independent clones (C8962, C8968, C8970, C8974, C9034 and C9036) were obtained (each considered having complete length of 5' terminus), and the nucleotide sequence of nt1-17 of the respective clones was determined.

The upstream sequence of strain HC-J1, in the region of nt18-843, was determined by clones C2503, C2508 and C2510 which were obtained by PCR amplification by the primers #44 (5'-GGCGACACTCCACCATGAAT-3') and #25 (5'-TCCCTGTTGCATAGTTCACG-3'). Nucleotide sequence of 5, terminus further upstream, nt1-37, was determined by 16 clones, C8931, C8932, C8935, C8937, C8942, C8944, C8949, C8950, C8951, C8954, C8955, C9023, C9026, C9030, C9031 and C9032 obtained by the same method as for the HC-J4 strain.

The downstream sequence of the 5' terminus from nt1984-2560 of HC-J1 was determined by 3 clones of C11444, C11450 and C11451 obtained by PCR amplification for 30 cycles by primers #148 (5'-TGCACCTGGATGAACTCAAC-3') and #146 (5'-AGTAGCATCATCCACAAGCA-3').

The downstream sequence from nt738 to 1900 of strain HC-J4 (having 1163 nucleotides) was determined by three clones of C2821, C3173 and C3192 which were obtained by PCR amplification by primers #30 (5'-CTCATGGGGTCACCACAAC-3'). and #42 (5'-TCGGTCGTCCCCACCACAAC-3').

Further downstream sequence from nt1860-2560 of strain HC-J4 was determined by 3 clones of C11462, C11463 and C11464 obtained by PCR amplification by primers #57 (5'-TATTGCTTCACCCCAAGCCC-3') and #146 above.

From the analysis described above, full nucleotide sequences of 5' termini of the genomes of HC-J1, HC-J4 and HC-J5 were determined as shown below.

From the analysis described above, full nucleotide sequences of 5' termini of genomes of HC-J1 and HC-J4 were determined as shown in figure 1 and figure 2 (see sequence lists 1 and 2).

5) Determination of 5' Terminal Nucleotide Sequence Of HC-J5 by Assembling cDNA Clones

The nucleotide sequence of the 5' terminus of strain HC-J5 was determined by analysis of clones obtained by PCR amplification as shown in figure 1(c).

Isolation of RNA from HC-J5, and determination of its sequence, was conducted in the same manner as described above.

Sequences in the range of nt21-1886 of the RNA were determined by respective clones obtained by amplification by PCR utilizing each pair of primers shown below:

nt24-265
#32(5'-ACTCCACCATAGATCACTCC-3')
#36 (5'-AACACTACTCGGCTAGCAGT-3')
Clones: C3534, C3536, C3537.
nt63-508
#33 (5'-TTCACGCAGAAAGCGTCTAG-3')
#51 (5'-GACCGCTCCGAAGTCTTCCT-3')
Clones: C4658, C4659, C4660.
nt467-843
#23 (5'-TAGATTGGGTGTGCGCGCGA-3')
#25 (5'-TCCCTGTTGCATAGTTCACG-3')
Clones: C2451, C2453, C2454.
nt732-934
#50 (5'-GCCGACCTCATGGGGTACAT-3')
#63 (5'-CTGGTGTTCTTAACCTGGAC-3')
Clones: C5592, C5593, C5594.
nt867-1354
#55 (5'-ATTTTCTTGCTGGCCCTGCT-3')
#54 (5'-ATCGCGTACGCCAGGATCAT-3')
Clones: C5164, C5303, C5331.
nt1240-1880
#38 (5'-TGCAATTGTTCTATCTACCC-3')
#41 (5'-CAGGGCTTGGGGGTGAAGCAA-3')
Clones: C3722, C3748, C3753.
nt1842-1976
#4 (5'-AGTGTGTGTGGTCCGGTATA-3')
#5 (5'-CGGTGGCCTGGTATTGTTAA-3')
Clones: C3952, C3969, C3990.

Three cDNA clones (C8995, C8997, C8998) were obtained in the same way as in example 1(4) to determine the nucleotide sequence from nt1-160 of further upstream of 5' terminus.

From the analysis described above, the full nucleotide sequence of 5' terminus of the genome of strain HC-J5 was determined, as shown in sequence list 3.

The non-coding region of the 5' terminus of HC-J5 was found to have 340 nucleotides, lacking a cytosine out of 5 cytosines from nt6-10 of HC-J1.

### 6) Determination of 5' Terminal Nucleotide Sequence Of HC-J6 by Assembling cDNA Clones

The nucleotide sequence of the 5' terminus of strain HC-J6 was determined by analysis of clones obtained by PCR amplification as shown in figure 1(d).

Isolation of RNA from HC-J6 and determination of its sequence was conducted in a manner as described above.

Sequences in the range of nt24-2551 of the RNA were determined by respective clones obtained by amplification by PCR utilizing each pair of primers shown below.

nt24-826
#32 (5'-ACTCCACCATAGATCACTCC-3')
#122 (5'-AGGTTCCCTGTTGCATAATT-3')
Clones: C9397, C9388, C9764
nt732-1907
#50 (5'-GCCGACCTCATGGGGTACAT-3')
#122 (5'-TCGGTCGTGCCCACTACCAC-3')
Clones: C9316, C9752, C9753
nt1847-2571
#149 (5'-TCTGTGTGTGGCCCAGTGTA-3')
#146 (5'-AGTAGCATCATCCACAAGCA-3')
Clones: C11621, C11624, C11655

Further upstream of the 5' terminus, from nt 1-160, was determined by the following 13 clones in the same way as example 1(4): C9577, C9579, C9581, C9587, C9590, C9591, C9595, C9606, C9609, C9615, C9616, C9619.

From the analysis described above, the full nucleotide sequence of the 5' terminus of the genome of strain HC-J6 was determined as shown in sequence list 4.

The noncoding region of the 5' terminus of HC-J6 was found to have 340 nucleotides lacking a cytosine out of 5 cytosines from nt6-10 of HC-J1.

### 7) Determination of 5' Terminal Nucleotide Sequence Of HC-J7 by Assembling cDNA Clones

The nucleotide sequence of the 5' terminus of strain HC-J7 was determined by analysis of clones obtained by PCR amplification as shown in figure 1(e).

Isolation of RNA from strain HC-J7 and determination of its sequence was made in a manner as described above. Sequences in the range of nt24-2498 of the RNA were determined by respective clones obtained by amplification by PCR utilizing each pair of primers shown below:

nt24-826
#32 (5'-ACTCCACCATAGATCACTCC-3')
#122 (5'-AGGTTCCCTGTTGCATAATT-3')
Clones: C10621, C10622, C10623.
nt732-1354
#50 (5'-GCCGATCTCATGGGGTACAT-3')
#54 (5'-ATCGCGTACGCCAGGATCAT-3')
Clones: C10461, C10463, C10615.
nt1309-1625
#129 (5'-CGCATGGCATGGGATATGAT-5')
#127 (5'-ATGTGCCAACTGCCGTTGGT-3')
Clones: C11183, C11184, C11185
nt1566-1888
#145 (5'-C AGGATATCAGTCTAATCAA-3')
#136 (5'-ACTGGGCTGGGAGTGAAACA-3')
Clones: C111361, C111364, C11374
nt1833-2518
#150 (5'-ATCGTCTCGGCTAAGACGGT-3')
#146 (5'-AGTAGCATCATCCACAAGCA-3')
Clones: C11535, C11540, C11566

Further upstream of the 5' terminus, from nt 1-160, was determined by the following 8 clones in the same way as example 1(4): C10513, C10515, C10521, C10554, C10558, C10568, C11231, C11232.

From the analysis described above, the full nucleotide sequence of the 5' terminus of the genome of strain HC-J7 was determined as shown in sequence list 5.

The 5' terminal sequences of HC-J1, HC-J4, HC-J5, HC-J6, and HC-J7 disclosed in the examples were different from the HCV sequence in European Patent Application Publication No. 388,232.

8. Determination of Amino Acid Sequences.

According to the 5' terminal nucleotide sequences of the genomes of strains HC-J1, HC-J4, HC-J5, HC-J6 and HC-J7, determination was made of the sequences of 733 amino acids (HC-J1 and HC-J4), 515 amino acids (HC-J5), 737 amino acids (HC-J6) and 719 amino acids (HC-J7) encoded by the structural gene region nt342-1880 (1545 nucleotides of the structural gene region beginning with ATG, excluding 340 or 341 nucleotides.

The amino acid sequences of the respective genomes are shown in sequence list 11 (HC-J1: P-733-1), sequence list 12 (HC-J4: P-733-2), sequence list 13 (HC-J5: 515), sequence list 14 (HC-J6: P-737) and sequence list 15 (HC-J7: P-719).

9. Determination of the Virus Core Regions.

There was 87.2% homology between the two sequences constituting the structural protein of strains HC-J1 and HC-J4. Homology between the sequences of the two genomes was 96.9% for amino acids 1-191 (core region) and 77.6% for amino acids 192-383 (envelope region).

All of the genomes of HC-J1, HC-J4, HC-J5, HC-J6 and HC-J7 showed higher homology in the upstream amino acids 1-191 (core region) than in the downstream amino acids 192-383 (envelope region). The upstream region was found to be highly conserved among HCV strains.

As regards hydrophilicity of amino acids 1-191, three highly hydrophilic parts were noted in amino acids 1-120 (figure 2). These parts were rich in basic amino acids such as arginine (28/120 = 23% in HC-J1) and the upstream was considered to code for the core protein of NANB hepatitis virus.

From the study of hydrophilicity scores, Applicants had presumed the sequence of 36 amino acids (CP-9) from 39 to 74 to form an epitope of the core protein. Amino acid sequence of CP-9 is shown in sequence list 22 (CP-9-1: HC-J1), sequence list 23 (CP-9-2: HC-J4) and sequence list 24 (CP-9-3: HC-J5, HC-J6, HC-J7). CP-9-1, -2, and -3 are generally called CP-9 below.

The nucleotide sequence of the gene coding for this amino acid sequence is shown in sequence list 29 (CN-9-1: HC-J1), sequence list 30 (CN-9-2: HC-J4), and sequence list 31 (CN-9-3: HC-J5). The CN-9 region of HC-J6 and HC-J7 is the sequence in sequence list 31 having partial displacement as far as without replacement of amino acids corresponding to 456-563 of the nucleotide sequence in sequence list 4 and 5. CN-9-1, -2, -3 are generally called CN-9 below.

In addition, CN-9-1 was used for CN-9 representing CN-9-2 and CN-9-3, and CP-9-1 was used for CP-9 representing CP-9-2 and CP-9-3 in the examples and experimental example below. The same results were obtained using CN-9-2, CN-9-3, CP-9-2 and CP-9-3. CN-5-1, -2, -3 are generally called CN-5 below. CP-9-1, -2, -3 are generally called CP-9 below.

While CP-9 was first obtained by chemical synthesis, CN-9 can produce CP-9 in host cells such as Escherichia coli by means of genetic engineering technique.

Determination of the epitope of the core protein of NANB hepatitis virus opened access to chemical synthesis of the peptide, manufacturing of the peptide by genetic engineering techniques, synthesis of the polynucleotides, manufacturing of the antibody, manufacturing of NANB hepatitis diagnostic reagents, and development of products such as NANB hepatitis vaccines.

Example 2.

Using the polypeptide CP-9 (amino acids 39-74) having the amino acid sequence shown in Example 1, the inventors established the following detection system for the antibody against NANB hepatitis virus.

1. Manufacture of Peptide.

According to the well-known method described by Merrifield, the inventors synthesized peptide CP-9-1 (sequence list 22) having the amino acid sequence of R R G P R L G V R A T R K T S E R S Q P R G R R Q P I P K V R R P E G R. After that, the product was hydrolyzed for 24 hours with 6N HCl under reduced pressure at $110\frac{1}{2}$C and its amino acid composition was confirmed.

Similarly, polypeptides CP-9-2 and CP-9-3 having sequences shown in sequence list 23 and 24 were synthesized.

Polypeptide CP-10 having the sequence shown in sequence list 21 was synthesized as well.

Polypeptides CP-5-1, CP-5-2 and CP-5-3 having the sequences shown in sequence list 25, 26 and 27 respectively were synthesized. CP-5-1, -2, and -3 are generally called CP-5 below.

CN-10 coding the amino acid sequence of CP-10 has the sequence shown in sequence list 28. CN-10 and polynucleotides having the sequence corresponding to nt354-410 in sequence list 1 to 4 are capable of expressing CP-10 in host cells by means of genetic engineering technique.

CN-5 coding the amino acid sequence of CP-5 has the sequence shown in sequence list 32 (CN-5-1: HC-J1, HC-J4), sequence list 33 (CN-5-2: HC-J5, HC-J6), sequence list 34 (CN-5-3; HC-J7). CN-10 and polynucleotides having the sequence corresponding to nt642-701 in sequence list 1 to 5 are capable of expressing CP-5 in host cells by means of genetic engineering technique.

CN-5 and CN-10 are capable of expressing CP-5 and CP-10 in host cells such as Escherichia coli by means of genetic engineering technique.

2. Detection System for Antibody Against NANB Hepatitis Virus.

The detection system was developed using polyvinyl microtiter plates and the sandwich method.

In this example, 50 Ìl of 5 Ìg/ml concentration of the peptide was dispensed in each well and incubated overnight at room temperature for consolidation. The microplate wells were washed five times with physiological saline containing 0.05% Tween 20. For overcoating, 100 Ìl of NaCl buffer containing 30% (v/v) of calf serum and 0.05% Tween 20 (CS buffer) was dispensed in each well and discarded after incubation for 30 minutes at room temperature.

For determination of samples, in the primary reaction, 50 Ìl of the CS buffer containing 30% calf serum and 10 Ìl of a sample was dispensed in each microplate well and incubated on a microplate vibrator for one hour at room temperature.

After completion of the reaction, microplate wells were washed five times in the same way as previously described.

In the secondary reaction, as labeled antibody 1 ng of horseradish peroxidase labeled anti-human IgG mouse monoclonal antibodies (Fab' fragment: 22G, Institute of Immunology Co., Ltd., Tokyo, Japan) dissolved in 50 Ìl of calf serum was dispensed in each microplate well, and was incubated on a microplate vibrator for one hour at room temperature. Wells were washed five times in the same way. After addition of hydrogen peroxide (as substrate) and 50 Ìl of O-phenylendiamine solution (as color developer) in each well, and after incubation for 30 minutes at room temperature, 50 Ìl of 4M sulphuric acid was dispensed in each well to stop further color development and for reading absorbance at 492 nm.

The cut-off level of this assay system was set by measuring a number of donor samples with normal ALT value of 34 Karmen unit or below and which tested negative for anti-HCV and its mean absorbance was plus 0.25 (0.05 + 0.25 = 0.30).

Example 3.

3' terminal nucleotide sequence and amino acid sequence of NANB hepatitis virus genome were determined in the following way:

(1) Determination of 3' Terminal Nucleotide Sequence By

Assembling cDNA Clones

As shown in figure 1(f), nucleotide sequences of 3' termini of genomes of strains HC-J1, HC-J4, HC-J5, HC-J6, and HC-J7 were determined by analysis of clones obtained by amplification of HCV specific cDNA by PCR.

The 938 nucleotide sequence of nt1-938 of strains HC-J1, HC-J4, HC-J5, HC-J6, and HC-J7 were determined by analysis of amplification products obtained by PCR using primer #80 (5'-GACACCCGCTGTTTTGACTC-3') and #60 (5'-GTTCTTACTGCCCAGTTGAA-3').

Obtained clones are shown below:

HC-J1: C7391, C7343, C7377;

HC-J4: C5584, C5585, C5586;

HC-J5: C7212, C7257, C7261;

HC-J6: C9760, C9234, C9761;

HC-J7: C9928, C11125, C11141.

Nucleotide sequences of 3' termini downstream from nt939 were determined in the manner shown below:

RNA was extracted from each sample in the manner described in example 1, followed by addition of poly(A) to 3' terminus of RNA using poly(A)-polymerase. cDNA was synthesized using oligo(dT)$_{20}$ as a primer, and used as a template in PCR.

1st stage PCR was performed using sense primer specific to each strain and oligo(dT)$_{20}$ as antisense primer. Then 2nd stage PCR was performed using cDNA obtained by 1st stage PCR using sense primer specific to each strain but downstream of that used in 1st stage PCR and oligo(dT)$_{20}$ as antisense primer. After smoothening both ends of PCR product obtained by two stage PCR utilizing T$_4$ DNA polymerase and phosphorylating the 5' terminus utilizing T$_4$ polynucleotide kinase, the nucleotide sequence was determined by subcloning to Hinc II site of M13mp19 phage vector.

Each pair of primer utilized in PCR and obtained clones are shown below:

HC-J1

#100 (5'-AAGGCTGCCATATGTGGCAA-3')

#91 (5'-GCCATATGTGGCAAGTACCT-3')

Clones: C9707, C9714, C9719, C9724, C9726, C9730, C9737, C9738, C9741, C9742, C9746, C9925, C9936, C9943, C9945, C9949.

HC-J4

#61 (5'-TTGCGAGTCTGGAGACATCG-3')

#78 (5'-TGTCCGCGCTAAGCTACTGT-3')

Clones: C8761, 8764, 8776, C8784, C8796, C8800, C8803, C8811, C8812, C8819, C8825, C8849, C8851.

HC-J5

#97 (5'AGTCAGGGCGTCCCTCATCT'3')

#90 (5'-GCCGTTTGCGGCCGATATCT-3')

Clones: C10820, C10827, 10829, 10843, 10848, 10849, 10864, 10865, 10872.

HC-J6

#97 (5'-AGTCAGGGCGTCCCTCATCT-3')

#90 (5'-GCCGTTTGCGGCCGATATCT-3')

Clones: C10820, C10827, C10829, C10843, C10844, C10848, C10849, C10864, C10865, C10872.

HC-J6

#97 (5'-AGTCAGGGCGTCCCTCATCT-3')

#90 (5'-GCCGTTTGCGGCCGATATCT-3')

Clones: C10311, 10313, C10314, C10320, C10322, C10323, C01326, C10328, C10330, C10333, C10334, C10336, C10337, C10345, C10346, C10347, C10349, C10350, C10357

HC-J7

#123 (5'CTTAGAGCGTGGAAGAGTCG-3')

#124 (5'-GCCATCTGTGGCCGTTACCT-3')

Clones: C10801, C10804, C10807, C10809, C10811, C10812, C10814, C10815, C10818

From the analysis described above, the full nucleotide sequences of 3' terminal sequences of strains HC-J1, HC-J4, HC-J5, HC-J6 and HC-J7 are shown in sequence sequence list 6-10.

The 3' terminal sequences of strains HC-J1, HC-J4, HC-J5, HC-J6 and HC-J7 disclosed in the examples, to a certain extent, overlapped HCV sequence of European Patent Application Publication No. 388,232 in nt 7938-8866 (929 nucleotides) but were different from it.

(2) Determination Of Amino Acids

According to the 3' terminal nucleotide sequences of the genomes of strains HC-J1, HC-J4, HC-J5, HC-J6 and HC-J7, determination was made of sequences of 365 amino acids encoded by the non-structural gene region of 1096 nucleotides, excluding 3' terminal non-coding region of 77 nucleotides.

The amino acid sequences of the respective genomes are shown in sequence list 16 (HC-J1: P-365-1), sequence list 17 (HC-J4: P-365-2), sequence list 18 (HC-J5: P-365-3), sequence list 19 (HC-J6: P-365-4) and sequence list 20 (HC-J7: P-365-5).

Experimental Example 1.

Five peptides (P19-36, P27-36, P19-27, P10-27 and P10-18) of different length in sequence relating to CP-9 (P39-74, 36 mer), considered to be constituting the epitope of the core region, were synthesized and their inhibition capability was tested.

In the test, 30 Ìl of the peptide (adjusted to 100 Ìl/mg; same volume for each sample) was mixed and measured after incubation for two hours at 37½C according to the method described in Example 1. Results are shown in Table 4.

Table 4. Epitope mapping on CP-9 by peptide-inhibition.

| Inhibition % | anti-CP-9 (+) | | |
|---|---|---|---|
| | 1 | 2 | 3 |
| CP9 | 100 | 97 | 96 |
| P19-36 | 80 | 91 | 49 |
| P27-36 | 63 | 82 | 36 |
| P19-27 | 51 | 71 | 20 |
| P10-27 | 39 | 52 | 40 |
| P10-18 | 31 | 33 | 21 |

From the inhibition rate of samples 1 and 2 in the test of positive anti-CP9 samples, it was confirmed that the CP-9 was antigenic throughout its sequence.

Experimental Example 2.

The detection system for antibody against NANB hepatitis virus developed and described in Example 2 was compared with the conventional anti-HCV detection kit by testing samples from patients with post-transfusion NANB hepatitis and patients with liver diseases. The results proved that the detection system under this invention was much superior to the conventional detection kit both in specificity and sensitivity for the acute phase of the disease.

1. Post-transfusion NANB Hepatitis.

This example concerns a patient who received seven units of blood (via transfusion over two times) and was infected with NANB hepatitis (which manifested itself about two months after transfusion). In this example, blood samples were retrospectively tested by the anti-HCV test kit and the kit developed under this invention; as shown in Table 1, one donor blood sample turned out to have an Absorbance over 2.000 and was positive.

Table 1.  Detection of anti-CP9 and anti-HCV in donor

bloods.

| | Antibody | |
|---|---|---|
| Donors | anti-CP9 | anti-HCV |
| No. 1 | 0.082 (-) | 0.018 (-) |
| No. 2 | >2.000 (+) | 0.006 (-) |
| No. 3 | 0.027 (-) | 0.007 (-) |
| No. 4 | 0.086 (-) | 0.063 (-) |
| No. 5 | 0.064 (-) | 0.025 (-) |
| No. 6 | 0.026 (-) | 0.005 (-) |
| No. 7 | 0.038 (-) | 0.009 (-) |

As to the recipient, the antibody detection system under the present invention showed absorbance in excess of 2 (coincidental to the abrupt rise in ALT levels of the patient two months after transfusion, while it was not until three months after the transfusion that the conventional test kit detected anti-HCV). The antibody detection system under the present invention thus proved its significant advantage over the conventional anti-HCV test kit both in specificity for the donor blood sample test and in earlier detection of the antibody in a patient sample.

2. Detection of Anti-CP9 in Sample from Patients with Liver Diseases.

Anti-CP9 was found in 13 cases out of 19 cases (68%) of sporadic acute NANB hepatitis and 15 cases out of 18 cases (83%) of post-transfusion NANB hepatitis.

In case of chronic NANB liver diseases, 103 cases out of 133 cases (77%) of patients with chronic hepatitis, 70 cases out of 113 cases (62%) of patients with liver cirrhosis, and 31 cases out of 41 cases (76%) of patients with hepatocellular carcinoma were found positive for anti-CP9.

On the other hand, only one case out of eight cases of acute hepatitis A patients was weakly positive and all of 11 cases of acute hepatitis B patients were negative for this antibody. 7% patients with chronic hepatitis, 12% of patients with liver cirrhosis, and 14% of patients with hepatocellular carcinoma of hepatitis B etiology were positive for this antibody.

12

All cases of auto-immune diseases, six cases of lupoid hepatitis, and nine cases of primary biliary liver cirrhosis were negative for this antibody.

In comparison of the above data with the data by Chiron Corporation (Science (1989), 244: 362-366) that 70.8% (17 cases out of 24 cases) of post-transfusion acute NANB hepatitis and 56.5% (34 cases out of 58 cases) of sporadic NANB hepatitis were positive for the anti-HCV antibody, it was concluded that anti-CP9 could be detected at a much superior and higher rate in acute hepatitis cases.

According to data on Japanese patients (Ortho Diagnostic Systems, Tokyo: Clinical Study), 2.4% (two cases out of 82 cases) of acute hepatitis B patients turned positive for anti-HCV, while, as described above, anti-CP9 under this invention did not cross-react with hepatitis B, thus proving high specificity.

3. Detection of Viral RNA in Samples with Overlapping or Discrepancy Between Ortho's Anti-HCV and Anti-CP9.

Test results by anti-HCV and anti-CP9 corresponded in 49% of acute NANB hepatitis (at the time of onset of the disease) and 66-67% of chronic hepatitis, liver cirrhosis and hepatocellular carcinoma.

Frequency of anti-CP9 in patients with various liver diseases are shown in Table 2. As shown in Table 2, the presence of HCV RNA was confirmed in 10 cases out of 11 cases of chronic NANB hepatitis patients testing negative for anti-HCV but positive for anti-CP9 (absorbance over 2).

Table 2.  Detection by PCR of viral RNA in samples from patients with NANB hepatitis related liver diseases positive for anti-CP9 but negative for anti-HCV.

| Sample | Diagnosis* | Age & Sex** | Absorbance at 492 nm | | HCV RNA (PCR) |
|--------|-----------|-------------|----------|----------|---------|
| | | | anti-CP9 | anti-HCV | |
| 1 | C H | 46M | >2.000 | 0.030 | + |
| 2 | C H | 45M | >2.000 | 0.076 | + |
| 3 | C H | 47M | >2.000 | 0.031 | + |
| 4 | C H | 60F | >2.000 | 0.163 | + |
| 5 | C H | 64F | >2.000 | 0.133 | + |

14

| | | | | | |
|---|---|---|---|---|---|
| 6 | L C | 47M | >2.000 | 0.253 | − |
| 7 | L C | 41F | >2.000 | 0.396 | + |
| 8 | L C | 62F | >2.000 | 0.393 | + |
| 9 | L C | 72M | >2.000 | 0.211 | + |
| 10 | L C | 56F | >2.000 | 0.126 | + |
| 11 | H C C | 52M | >2.000 | 0.061 | + |

\* CH = Chronic Hepatitis
\* LC = Liver Cirrhosis
\*\* HCC = Hepatocellular Carcinoma
\*\* F = Female
\*\* M = Male

From the above results, it was confirmed again that anti-CP9 had higher sensitivity and specificity in diagnosis of chronic NANB hepatitis.

4. Comparison in Detection of Antibody in Acute NANB Hepatitis Samples.

Of three post-transfusion NANB hepatitis and four sporadic NANB hepatitis cases, samples taken at the time of onset of the disease (0 month), three months later and six months later, and were tested for anti-CP9 and anti-HCV to compare timing of their sero-conversion. Results are shown in Table 3.

Table 3.  Detection of anti-CP9 and anti-HCV in post-

transfusion and sporadic NANB hepatitis patients.

anti-CP9 (upper) and anti-HCV (lower) months after manifestation of disease. Figures are Absorbance.

| Infection Source | Sample No. Age & Sex | 0 Month | 3 Months | 6 Months |
|---|---|---|---|---|
| TRANSFUSION | 1  37F | >2.000 | >2.000 | >2.000 |
|  |  | 0.103 | 0.874 | 0.287 |
|  | 2  65M | 1.271 | 1.244 | 0.813 |
|  |  | 0.109 | 1.106 | >2.000 |
|  | 3  65M | >2.000 | >2.000 | >2.000 |
|  |  | 0.085 | 0.897 | 0.392 |
| SPORADIC | 1  23M | 0.758 | >2.000 | >2.000 |
|  |  | 0.103 | 0.476 | 1.078 |
|  | 2  43M | >2.000 | >2.000 | >2.000 |
|  |  | 1.531 | >2.000 | >2.000 |

```
¤         D        °   3    °  26M   °  >2.000      >2.000      >2.000
¤
¤         I        °        °        °
¤
¤         C        °        °        °   0.093      1.451       1.555
¤
¤
ûáááááá6áááááá6ááááááááááááááááááááááááááááááááááÑ
¤                 °   4    °  26M   °  >2.000      >2.000      >2.000
¤
¤                 °        °        °
¤
¤                 °        °        °   0.062      0.075       1.196
¤
àëëëëëëëëëëëüëëëëëëüëëëëëëëüëëëëëëëëëëëëëëëëëëëëëëëëëëëëë
ëë¥
```

For all of the three post-transfusion NANB hepatitis and four sporadic NANB hepatitis cases, anti-CP9 was positive at the time of onset of the disease, while only one case out of seven cases (sporadic hepatitis) was positive for anti-HCV at the time of onset of the disease, five cases turned positive after three months and the remaining one case turned positive as late as six months after onset of the disease. This confirms that the test for anti-CP9 is capable of detecting specific antibody appearing in an early stage of acute NANB hepatitis much faster than anti-HCV.

From the above, it can be said that without relying upon the conventional diagnostic method, anti-CP9 has made it possible to diagnose acute NANB hepatitis.

Experimental Example 3.

An example of determination of NANB hepatitis virus antibody by peptide having the amino acid sequence of CP-10 (P5-23, 19 mer) is shown below. Determination method is same as that shown in Example 2.

Anti-CP9 and anti-CP10 showed similar frequency (Table 5), however, as for the sample No. 6, it was positive for anti-CP10 while it was negative for anti-CP9, suggesting that anti-CP10 was complementary to anti-CP9.

Table 5. Detection of anti-CP9, anti-HCV and HCV-RNA in patients with chronic NANB hepatitis positive for anti-CP10.

| | | | | Absorbance (A492) | | | |
|---|---|---|---|---|---|---|---|
| Sample No. | Diagnosis* | Age & Sex** | anti-CP10 | anti-CP9 | anti-HCV | HCV-RNA (PCR) |
| 1 | C H | 46M | >2.000 | >2.000 | 0.031 | + |
| 2 | C H | 44M | >2.000 | >2.000 | 0.076 | + |
| 3 | H C C | 51M | >2.000 | >2.000 | 0.065 | + |
| 4 | C H | 45M | 1.024 | >2.000 | 0.030 | + |
| 5 | L C | 55F | 0.980 | >2.000 | 0.126 | + |
| 6 | L C | 60M | >2.000 | 0.164 | >2.000 | + |
| 7 | L C | 69M | 1.860 | 1.834 | >2.000 | + |
| 8 | L C | 61M | 1.288 | >2.000 | >2.000 | + |
| 9 | H C C | 65M | 1.345 | >2.000 | 1.213 | + |
| 10 | L C | 54M | 1.196 | >2.000 | >2.000 | + |

* CH = Chronic Hepatitis

```
*
* LC = Liver Cirrhosis
*
** HCC = Hepatocellular Carcinoma
** F = Female
  M = Male
```

Samples Nos. 1 to 5 were negative for anti-HCV (Chiron Corp.) but were positive for anti-CP10 and anti-CP9, and NANB hepatitis viral RNA was detected by PCR in all of them. Thus the detections systems using anti-CP10 is superior to anti-HCV test kit both in sensitivity and specificity.

Experimental Example 4.

An example of determination of NANB hepatitis viral antibody using peptide having the amino acid sequence of CP-5 (P101-120, 20 mer) is shown below. Determination method is same as that shown in Example 2.

When anti-CP5 and anti-CP9 were compared, anti-CP9 showed higher frequency than anti-CP5 in a series of samples. However, anti-CP5 was positive for sample Nos. 8 to 10 for which anti-CP9 was negative and was considered to be complementary to anti-CP9. Sample Nos. 5 to 7 were negative for anti-HCV but positive for anti-CP5 and anti-CP9 and presence of NANB hepatitis viral RNA was confirmed by PCR. This proved higher specificity and sensitivity of the detection system using CP-5 over anti-HCV detection kit (Table 6).

Table 6. Determination of anti-CP9, anti-HCV and HCV-RNA in patients with chronic NANB hepatitis positive for anti-CP5.

| Sample No. | Diagnosis[*] | Age & Sex[**] | Absorbance (A492) | | | HCV-RNA (PCR) |
|---|---|---|---|---|---|---|
| | | | anti-CP5 | anti-CP9 | anti-HCV | |
| 1 | C H | 55M | >2.000 | >2.000 | >2.000 | + |
| 2 | C H | 65M | >2.000 | >2.000 | >2.000 | + |
| 3 | C H | 65F | >2.000 | >2.000 | >2.000 | + |
| 4 | L C | 66F | >2.000 | >2.000 | >2.000 | + |
| 5 | C H | 66F | >2.000 | >2.000 | 0.183 | + |
| 6 | C H | 61F | 0.934 | >2.000 | 0.163 | + |
| 7 | L C | 63M | 1.052 | >2.000 | 0.403 | + |
| 8 | L C | 63M | 1.688 | 0.249 | >2.000 | + |
| 9 | C H | 58M | 0.639 | 0.252 | >2.000 | + |
| 10 | L C | 49M | 1.174 | 0.241 | 0.391 | + |

```
*
* CH = Chronic Hepatitis
*
* LC = Liver Cirrhosis
*
**HCC = Hepatocellular Carcinoma
** F = Female
  M = Male
```

This invention makes possible detection of NANB hepatitis virus infection which could not be detected by conventional determination methods, and provide NANB hepatitis detection kits capable of highly specific and sensitive detection at an early phase of infection.

These features allow accurate diagnosis of patients at an early stage of the disease and also help to remove at higher rate NANB hepatitis virus carrier bloods through screening test of donor bloods.

Polypeptides and their antibodies under this invention can be utilized for manufacture of vaccines and immunological pharmaceuticals, and structural gene of NANB hepatitis virus provides indispensable tools for detection of polypeptide antigens and antibodies.

Antigen-antibody complexes can be detected by methods known in this art. Specific monoclonal and polyclonal antibodies can be obtained by immunizing such animals as mice, guinea pigs, rabbits, goats and horses with NANB peptides (e.g., bearing NANB hepatitis antigenic epitope described in the above examples).

Further variations and modifications of the invention will become apparent to those skilled in the art from the foregoing and are intended to be encompassed by the claims appended hereto.

Japanese Priority Application 196175/91, filed April 10, 1991, is relied on and incorporated by reference. U.S. patent applications serial no. 07/540,604 (filed June 19, 1990), 07/653,090 (filed February 8, 1991), and 07/712,875 (filed June 11, 1991) are incorporated by reference in their entirety.

**Claims**

1. Recombinant cDNA of non-A, non-B hepatitis virus, strain HC-J1, comprising the nucleotide sequence of sequence list 1.

2. Recombinant cDNA of non-A, non-B hepatitis virus, strain HC-J4, comprising the nucleotide sequence of sequence list 2.

3. Recombinant cDNA of non-A, non-B hepatitis virus, strain HC-J6, comprising the nucleotide sequence of sequence list 4.

4. Recombinant cDNA of non-A, non-B hepatitis virus, strain HC-J7, comprising the nucleotide sequence of sequence list 5.

5. Recombinant cDNA of non-A, non-B hepatitis virus, strain HC-J1, comprising the nucleotide sequence of sequence list 6.

6. Recombinant cDNA of non-A, non-B hepatitis virus, strain HC-J4, comprising the nucleotide sequence of sequence list 7.

7. Recombinant cDNA of non-A, non-B hepatitis virus, strain HC-J5, comprising the nucleotide sequence of sequence list 8.

8. Recombinant cDNA of non-A, non-B hepatitis virus, strain HC-J6, comprising the nucleotide sequence of sequence list 9.

9. Recombinant cDNA of non-A, non-B hepatitis virus, strain HC-J7, comprising the nucleotide sequence of sequence list 10.

10. Amino acid sequence corresponding to recombinant cDNA of non-A, non-B hepatitis virus, strain HC-J1, comprising the amino acid sequence of sequence list 11.

11. Amino acid sequence corresponding to recombinant cDNA of non-A, non-B hepatitis virus, strain HC-J4, comprising the amino acid sequence of sequence list 12.

12. Amino acid sequence corresponding to recombinant cDNA of non-A, non-B hepatitis virus, strain HC-J6, comprising the amino acid sequence of sequence list 14.

13. Amino acid sequence corresponding to recombinant cDNA of non-A, non-B hepatitis virus, strain HC-J7, comprising the amino acid sequence of sequence list 15.

14. Amino acid sequence corresponding to recombinant cDNA of non-A, non-B hepatitis virus, strain HC-J1, comprising the amino acid sequence of sequence list 16.

15. Amino acid sequence corresponding to recombinant cDNA of non-A, non-B hepatitis virus, strain HC-J4, comprising the amino acid sequence of sequence list 17.

16. Amino acid sequence corresponding to recombinant cDNA of non-A, non-B hepatitis virus, strain HC-J5, comprising the amino acid sequence of sequence list 18.

17. Amino acid sequence corresponding to recombinant cDNA of non-A, non-B hepatitis virus, strain HC-J6, comprising the amino acid sequence of sequence list 19.

18. Amino acid sequence corresponding to recombinant cDNA of non-A, non-B hepatitis virus, strain HC-J7, comprising the amino acid sequence of sequence list 20.

19. Recombinant peptide CP-9-2 of non-A, non-B hepatitis virus, strain HC-J4, comprising the peptide sequence of sequence list 23.

20. Recombinant peptide CP-9-3 of non-A, non-B hepatitis virus, strains HC-J5, HC-J6 and HC-J7, comprising the peptide sequence of sequence list 24.

21. Recombinant polynucleotide CN-9-2 of non-A, non-B hepatitis virus, strain HC-J4, comprising the DNA sequence of sequence list 30.

22. Recombinant polynucleotide CN-9-3 of non-A, non-B hepatitis virus, strains HC-J5, HC-J6, and HC-J7, comprising the DNA sequence of sequence list 31.

23. Recombinant peptide CP-5-2 of non-A, non-B hepatitis virus, strains HC-J5 and HC-J6, comprising the peptide sequence of sequence list 26.

24. Recombinant peptide CP-5-3 of non-A, non-B hepatitis virus, strain HC-J7, comprising the peptide sequence of sequence list 27.

25. Recombinant polynucleotide CN-5-2 of non-A, non-B hepatitis virus, strains HC-J5 and HC-J6, comprising the DNA sequence of sequence list 33.

26. Recombinant polynucleotide CN-5-3 of non-A, non-B hepatitis virus, strain HC-J7, comprising the DNA sequence of sequence list 34.

27. A non-A, non-B hepatitis diagnostic test kit for analyzing samples for the presence of antibodies directed against a non-A, non-B hepatitis antigen, comprising the amino acid sequence according to sequence lists 23, 24, 26 or 27 as antigen attached to a solid substrate and labeled anti-human immunoglobulin.

28. A method of detecting antibodies directed against a non-A, non-B hepatitis antigen in a sample, said method comprising:
   (a) reacting said sample with the amino acid sequence according to sequence lists 23, 24, 26 or 27 to form antigen-antibody complexes; and
   (b) detecting said antigen-antibody complexes.

22

29. A non-A, non-B hepatitis specific monoclonal or polyclonal antibody reactive with an antigen, said antigen comprising the amino acid sequence according to sequence lists 23, 24, 26 or 27.

30. A method of detecting non-A, non-B hepatitis antigen in a sample, said method comprising:
(a) reacting said sample with the non-A, non-B hepatitis monoclonal or polyclonal antibody according to claim 29 to form antigen-antibody complexes; and
(b) detecting said antigen-antibody complexes.

31. A nucleotide sequence having substantially the sequence of genomes of NANB hepatitis virus strains HC-J1, HC-J4, HC-J5, HC-J6 or HC-J7 according to claims 1-9 and effective mutants and variants thereof, wherein one or more oligonucleotide fragments thereof are effective in highly sensitive detection of NANB hepatitis virus.